# EUROPEAN PATENT APPLICATION

(11) **EP 4 768 084 A1**
(43) Date of publication of application: **01.07.2026**
(21) Application number: 23955416.5
(22) Date of filing: 11.10.2023
(51) Int. Cl.: A61Q 1/00, A61K 8/06, A61K 8/19, A61K 8/29

(54) **OILY OR EMULSIFIED MAKEUP COSMETIC**

(71) Applicant: Nippon Shikizai, Inc., Minato-ku Tokyo 108-0073 (JP)
(72) Inventor: YAMAGUCHI, Kuzuhiro, Tokyo 1080073 (JP)
(74) Representative: HGF
(86) International application number: PCT/JP2023/036853
(87) International publication number: WO 2025/079165

(57) **Abstract**

The present invention provides an oily or emulsified makeup cosmetic that is free of ultrafine particles of concern for human health, and microplastic beads and volatile cyclic silicones of environmental concern, and is excellent in stability and usability. The oily or emulsified makeup cosmetic contains (A) metal oxide particles having a minor axis D50 of 90 to 140 nm, and a minor axis D90/D10 of 2.1 or less; and (B) a clay mineral, and is free of microplastic beads and volatile cyclic silicones.

## Description

### TECHNICAL FIELD

The present invention relates to oily or emulsified makeup cosmetics. More particularly, the present invention relates to oily or emulsified makeup cosmetics that are produced in consideration of the environment and safety, and are uniform in finish and excellent in stability.

### BACKGROUND ART

Conventionally, ultrafine particle titanium dioxide and ultrafine particle zinc oxide having an average particle size of 50 nm or less have been used as ultraviolet light scattering agents due to their high ultraviolet protection effect. Cosmetics containing ultrafine particle titanium dioxide having an average particle size of 50 nm or less and volatile cyclic silicone are known (see, for example, Patent Documents 1 and 2). Volatile cyclic silicones and microplastic beads are useful for the purpose of ensuring a uniform finish and reducing unnatural gloss. However, in recent years, with the growing global interest in environmental issues, environmental contamination problems associated with volatile cyclic silicones and microplastic beads have become apparent.

A water-in-oil type emulsified cosmetic is known which contains, based on the total amount of the cosmetic, (A) 5 to 15% by mass of an oil-soluble film-forming agent, (B) 0.1 to 5% by mass of a glycerin fatty acid ester, and (C) 20 to 50% by mass of a volatile linear silicone oil (see, for example, Patent Document 3). In the invention disclosed in Patent Document 3, the components (B) and (C) used instead of volatile cyclic silicones achieve both the stability and good spreadability of the cosmetic.

There is a known water-in-oil type emulsified cosmetic that contains, in an oil phase containing a volatile oil, (A) a solid oil powder, (B) an emulsifying silicone elastomer, and (C) titanium dioxide, in which the volatile oil is a hydrocarbon oil or an non-cyclic silicone (see, for example, Patent Document 4).

### REFERENCE DOCUMENT LIST

### PATENT DOCUMENTS

Patent Document 1: JP 2011-231102 A
Patent Document 2: JP 2019-6714 A
Patent Document 3: JP 2022-177984 A
Patent Document 4: JP 2021-50169 A

### SUMMARY OF THE INVENTION

### PROBLEM TO BE SOLVED BY THE INVENTION

In recent years, titanium dioxide particles having a particle size of 60 nm or less have been reported to cause inflammation when entering a living body, which has become a safety concern. Therefore, there is a tendency among sunscreen users, particularly consumers in Europe, to avoid blending titanium dioxide particles with a particle size of 60 nm or less in cosmetics. Furthermore, makeup cosmetics containing a large amount of such a titanium dioxide with a small particle size have a problem that an unnatural and unpleasant glossy texture is caused, and when applied, the cosmetics sink into the unevenness of the skin, resulting in a non-uniform finish. In addition, in order to reduce the environmental load, the market is demanding that cosmetics be free of microplastic beads and volatile cyclic silicones.

### MEANS FOR SOLVING THE PROBLEM

As a result of extensive investigation, the present inventor has conceived that, by using metal oxide particles having a prescribed particle size distribution, it is possible to obtain a cosmetic excellent in feel upon use and stability without using components conventionally used, and thus have completed the present invention.

According to one embodiment, the present invention relates to an oily or emulsified makeup cosmetic that contains (A) metal oxide particles having a minor axis D50 of 90 to 140 nm and a minor axis D90/D10 of 2.1 or less, and (B) a clay mineral, and is free of microplastic beads and volatile cyclic silicones.

In the oily or emulsified makeup cosmetic, the metal oxide particles are preferably contained in an amount of 2 to 30% by mass relative to 100% by mass of the total amount of the makeup cosmetic, and the metal oxide particles are preferably coated metal oxide particles that are coated with a hydrophobic treatment agent selected from the group consisting of fatty acid soap, alkoxysilanes, dimethicone, methylhydrogensiloxane, an acylamino acid, and a sugar fatty acid ester.

In the oily or emulsified makeup cosmetic, the clay mineral is preferably contained in an amount of 0.25 to 5% relative to 100% of the total amount of the makeup cosmetic.

The oily or emulsified makeup cosmetic preferably further contains (C) an iron oxide selected from yellow, red, and black iron oxides.

The oily or emulsified makeup cosmetic is preferably an oily or water-in-oil type makeup cosmetic.

The oily or emulsified makeup cosmetic preferably further contains a white pigment titanium dioxide, and the white pigment titanium dioxide is preferably contained in an amount of 5% by mass or less relative to 100% by mass of the total amount of the makeup cosmetic.

### EFFECTS OF THE INVENTION

The present invention can provide an oily or emulsified makeup cosmetic that is free of metal oxides having small particle sizes that raise safety concerns, and microplastics and volatile cyclic silicones that are hardly decomposable in the environment, and that is excellent in stability over time, spreadability, finish uniformity, and matte texture on the skin.

### MODE FOR CARRYING OUT THE INVENTION

In one embodiment, the present invention relates to an oily or emulsified makeup cosmetic. The makeup cosmetic of the present invention is a cosmetic that aesthetically adjusts the surface condition of keratin of the skin or hair, and in the present invention, refers to a cosmetic that adjusts light reflection and color tone by using a metal oxide powder. An oily cosmetic refers to a cosmetic in which a powder base is dispersed in an oily base of a fat or oil, a wax, or the like that is in the form of a liquid, a paste, or a solid at room temperature (1 to 30°C). An emulsified cosmetic refers to a cosmetic that is in the form of a liquid, a cream, or a solid at room temperature, and may be either a water-in-oil (W/O) type or an oil-in-water (O/W) type, and is an emulsion in which an aqueous component and an oily component are emulsified by an emulsifier. Hereinafter, the oily or emulsified makeup cosmetic may sometimes be simply referred to as the "cosmetic ".

The cosmetic of the present invention is a cosmetic that contains components (A) and (B) described below as essential components, and may further contain an optional component. Furthermore, the cosmetic of the present invention contains neither microplastic beads nor volatile cyclic silicones.

"Microplastic beads (MPB)", in the narrow sense, are understood as "synthetic, non-water-soluble solid plastic particles, less than 5 mm in size, intentionally blended in personal care products for the purpose of exfoliation or cleansing". The term "MPB" as used herein includes, in addition to the narrowly defined MPB, solid spherical particles of organic synthetic polymer less than 5 mm in size that have been conventionally blended in cosmetics for the purpose of improving usability, such as powders or fibers of nylon, acrylic acid esters, polyethylene, polystyrene, polyethylene terephthalate, polyurethane, organopolysiloxanes, and partially cross-linked organopolysiloxanes. Naturally derived polymers such as cellulose, and inorganic polymers such as silica, do not fall under the definition of MPB.

Volatile cyclic silicones are cyclic molecules that do not exist in nature and have a cyclic Si-O structure. Cyclic silicones are also referred to as cyclomethicones. The volatile cyclic silicones not contained in the cosmetic of the present invention are cyclic compounds having 3 or more and 6 or less Si atoms constituting a siloxane structure. Specific examples include hexamethylcyclotrisiloxane, octamethylcyclotetrasiloxane , decamethylcyclopentasilox ane, dodecamethylcyclohexasiloxane, and tetradecamethylcycloheptasiloxane, and all of the volatile cyclic silicones that satisfy the aforementioned definition of the number of Si atoms shall not be contained in the cosmetic of the present invention. Examples of commercially available products include, as decamethylcyclopentasiloxane, Excecall D-5(manufactured by Shin-Etsu Chemical Co., Ltd.), SH245 and DC345 (both manufactured by Dow Toray Co., Ltd.), and as dodecamethylcyclohexasiloxane, DC246 (manufactured by Dow Corning Toray Silicone Co., Ltd.).

In the cosmetic of the present invention, particles having a minor axis length of 100 nm or less preferably constitute 50% by mass or less. The particles here mean all the particle components including the essential component A, described later, and all optionally includable particle components. This is to avoid nanoparticles and ensure safety for living bodies, even when optional fine particles are contained in addition to the component A. More specifically, when particles having a minor axis length of 60 nm or less are contained, the content of such particles is preferably 5% by mass at maximum, and more preferably less than 1% by mass.

Now, the respective components will be described.

### (A) Metal oxide particles having minor axis D50 of 90 to 140 nm and minor axis D90/D10 of 2.1 or less

The component (A) is a group of metal oxide particles having a prescribed particle size and a particle size distribution, and is a group of metal oxide particles that are white under visible light (wavelength of 380 nm to 780 nm). Specifically, the component (A) is a group of metal oxide particles having a minor axis median diameter (D50) of 90 to 140 nm and a minor axis D90/D10 of 2.1 or less. D10 refers to the minor-axis length at 10% cumulative fraction from the fine-particle side of a cumulative particle size distribution of the group of metal oxide particles. D90 refers to the minor-axis length at 90% cumulative fraction from the fine-particle side of the cumulative particle size distribution of the group of metal oxide particles. Herein, unless otherwise specified as referring to the major axis, D10, D50, and D90 each denote values based on the minor axis length. In addition, herein, a group of metal oxide particles that satisfies the conditions of the component (A) is referred to as "metal oxide fine particles". The shape of the metal oxide fine particles is not particularly limited, and may be, for example, barrel-shaped, spindle-shaped, or spherical, and the major axis length may be approximately three times or less the minor axis length. Accordingly, the major and minor axis lengths may be nearly the same in some cases.

The metal oxide fine particles of the present invention are advantageous in that, when blended in a cosmetic, they maintain ultraviolet protection effect while exhibiting low safety concerns, and provide a matte texture without imparting an unnatural gloss. When the D50 is less than 90 nm, ultrafine particles having a particle size of 60 nm or less are included in a large amount, raising concerns regarding safety for the human body. In addition, a cosmetic containing many ultrafine particles is disadvantageous because it exhibits an unnatural gloss, has reduced matte texture, and is prone to becoming non-uniform. Also, when the D50 exceeds 140 nm and is less than 200 nm, the cosmetic tends to become non-uniform and the stability is disadvantageously reduced. The minor axis D50 is preferably 95 to 130 nm, and preferably 100 to 115 nm. In addition, the minor axis D90/D10 is preferably 2.1 or less, more preferably 1.8 or less, and further preferably 1.4 or less.

A compound constituting the metal oxide fine particles that are white under visible light may be, for example, titanium dioxide (TiO₂), zinc oxide (ZnO), aluminum oxide (Al₂O₃), magnesium oxide (MgO), and/or cerium oxide (CeO₂). In particular, TiO₂, ZnO, or a mixture of these is preferred. An iron oxide does not fall under the definition of the metal oxides of the component A.

The metal oxide fine particles may be uncoated particles composed substantially only of metal oxides. Alternatively, the metal oxide fine particles may be particles coated with a surface treatment agent. The surface treatment agent may be an inorganic compound such as aluminum hydroxide, or may be a hydrophobic treatment agent containing an organic component. Metal oxide fine particles coated with a hydrophobic treatment agent may be advantageously used in the oily or emulsified cosmetic because such particles can suppress the localized accumulation of oily components in the skin depression, and can promote formation of a uniform oily film of the cosmetic. As the hydrophobic treatment agent, one or more compounds selected from the group consisting of fatty acid soap, alkoxysilane, dimethicone, methylhydrogensiloxane, acylamino acid, and a sugar fatty acid ester may be used. Furthermore, by using a surface treatment agent such as a hydrophobic treatment agent, at least a portion of the surface of the powder, preferably the entire surface of the powder, can be coated.

As the fatty acid soap, that is, one of hydrophobic treatment agents, aluminum stearate hydroxide, aluminum stearate, magnesium stearate, and isopropyl titanium triisostearate are preferred, as the alkoxysilane, triethoxycaprylylsilane and trihexylsilane are preferred, and as the sugar fatty acid ester, dextrin stearate and dextrin isostearate are preferred, but the treatment agent is not limited thereto.

The acylamino acid is preferably selected from N-acylamino acids having a hydrocarbon group with 8 to 22 carbon atoms or salts thereof, and fatty acids or salts thereof, and specific examples include the following. Examples of N-acylamino acids or salts thereof include lauroyl lysine and N-acyl glutamate salts (such as sodium N-lauroyl glutamate, disodium N-stearoyl glutamate, and sodium N-myristoyl-L-glutamate). Among these, stearoyl glutamate salts and cocoyl glutamate salts are preferred. Examples of the fatty acid include palmitic acid, isostearic acid, stearic acid, lauric acid, myristic acid, behenic acid, oleic acid, rosin acid, and 12-hydroxystearic acid.

The content of the metal oxide fine particles of the component (A), whether uncoated or coated with a surface treatment agent, is preferably 2 to 30% by mass based on the total mass of the cosmetic taken as 100%. When the content of the metal oxide fine particles is less than 2% by mass, the uniformity and matte texture of the cosmetic may be reduced in some cases. When the content exceeds 30% by mass, the stability of the cosmetic may be reduced, and the cosmetic may be heavily spread (poor in spreadability) upon application. The content of the metal oxide fine particles is more preferably 5.5 to 24% by mass. When the content falls in this range, the stability, spreadability, matte texture, and uniformity are further improved. The content of the metal oxide fine particles is further preferably 11 to 20% by mass. When the content falls in this range, a cosmetic having the most favorable balance of stability, spreadability, matte texture, and uniformity can be obtained.

In a preferred embodiment, the metal oxide fine particles may contain a mixture of TiO₂ and ZnO. In such a mixture, it is sufficient that the D50 and the D10/D90 fall within the ranges described above. In such a case, a preferred blending amount of the mixture is within the range described above, and a mixing ratio (mass ratio) of TiO₂ and ZnO is not particularly limited and may be any ratio. For example, the mass ratio of TiO₂ and ZnO may be from 1:9 to 9:1, and preferably from 1:4 to 2:1, but is not limited to these ranges.

### (B) Clay mineral

The clay mineral is contained as an essential component of the cosmetic, and functions both to stabilize the cosmetic and to impart a favorable matte texture. The clay mineral refers to a type of colloidal hydrated aluminum silicate having a three-layer structure, and a compound represented by the following general formula (1), or a modified product thereof, can be used.

(X, Y)ₐ(Si, Al)₄O₁₀(OH)_{2-b}(F)_{b}Z_{1/3}·nH₂O (1)

wherein X represents one or more selected from Al, Fe (III), Mn (III), and Cr (III), or may be absent,
Y represents one or more selected from Mg, Fe (II), Ni, Zn, and Li, or may be absent,
Z represents one or more selected from K, Na, and Ca, or may be absent, and
a is 2 to 3, and b is 0 to 2.

In the formula (1), (X, Y) indicates that X and Y are present in a mixed state within a mineral crystal. Accordingly, (Si, Al) similarly indicates that Si and Al are present in a mixed state within a mineral crystal.

Specific examples of the clay mineral include a group of natural or synthetic montmorillonites (also referred to as smectites), such as montmorillonite, saponite, and hectorite, and synthetic micas such as sodium silicic mica and those known as sodium or lithium taeniolite. Examples of commercially available products include, but are not limited to, Kunipia and Smecton (manufactured by Kunimine Industries Co., Ltd.), Bengel (manufactured by Nihon Yuukinendo Co., Ltd.), Laponite (manufactured by BYK Japan KK), and Dimonite (manufactured by Topy Industries, Ltd.).

In addition, organically modified clay minerals obtained by cation-exchanging these materials with a quaternary ammonium salt cationic surfactant are also herein referred to as the clay minerals. For example, a clay mineral obtained by modifying a compound represented by the above formula with a quaternary ammonium salt cationic surfactant is a representative example, but the invention is not limited thereto. Specific examples include organically modified smectites such as dimethyldistearylammonium hectorite (disteardimonium hectorite), benzyldimethylstearylammonium hectorite, dimethyldistearylammonium bentonite, and magnesium aluminum silicate treated with distearyldimethylammonium chloride. Examples of commercially available products include Bentone 27 and Bentone 38 (disteardimonium hectorite; manufactured by Elementis Japan), Esben W (quaternium-18 bentonite; manufactured by Nihon Yuukinendo Co., Ltd.), and Esben WV (quaternium-90 bentonite; manufactured by Nihon Yuukinendo Co., Ltd.). Among these, dimethylalkylammonium hectorite, quaternium-18 bentonite, and quaternium-90 bentonite are preferred. In an oily or W/O type emulsified cosmetic, it is preferable to use an organically modified clay mineral, whereas in an O/W type emulsified cosmetic, it is preferable to use a clay mineral that is not organically modified.

The clay mineral may be a single type or a mixture of two or more types. In an oily or W/O type emulsified cosmetic, the content of the clay mineral as the component (B) is preferably 0.25 to 5% by mass based on the total mass of the cosmetic taken as 100%. When the content of the clay mineral is less than 0.25% by mass, the uniformity and stability of the cosmetic may be reduced in some cases. Also, when the content exceeds 5% by mass, the uniformity and stability of the cosmetic may be reduced, and the matte texture of the cosmetic may be reduced in some cases. The content of the clay mineral is more preferably 0.3 to 3% by mass. When the content falls in this range, the uniformity and stability, and the matte texture obtained upon application are further improved. The content of the clay mineral is further preferably 0.6 to 2.5% by mass. When the content falls in this range, a cosmetic well balanced in all of uniformity, stability, and matte texture upon application can be obtained.

In an O/W type emulsified cosmetic, the content of the clay mineral as the component (B) is preferably 0.6 to 5% by mass based on the total mass of the cosmetic taken as 100%. When the content of the clay mineral is less than 0.6% by mass, the uniformity and stability of the cosmetic may be deteriorated in some cases. Also, when the content exceeds 5% by mass, the uniformity and stability of the cosmetic may be reduced, and the matte texture of the cosmetic may be reduced in some cases. The content of the clay mineral is more preferably 0.7 to 3% by mass. When the content falls in this range, the uniformity and stability, and the matte texture obtained upon application are further improved. The content of the clay mineral is further preferably 0.8 to 2.5% by mass. When the content falls in this range, a cosmetic well balanced in all of uniformity, stability, and matte texture upon application can be obtained.

By containing these essential components (A) and (B), a cosmetic of the present invention can be obtained, and in particular, a cosmetic exhibiting good stability and spreadability, and excellent in matte texture, with a moderately suppressed gloss, can be obtained without using a component having a particle size of 60 nm or less that may pose risks to the human body, and microplastic beads or volatile cyclic silicones that may adversely affect the environment. Components that may optionally be contained will now be described.

### (C) Iron oxide

An iron oxide is a powder component that functions as a coloring component in a cosmetic. One or more types of iron oxides selected from yellow iron oxide (iron oxide yellow), red iron oxide (iron oxide red), and black iron oxide (iron oxide black) may be used, and an appropriate combination of these can be used with a mixing ratio of these three iron oxides changed according to a desired coloration of the cosmetic. The iron oxide may be uncoated particles composed substantially only of iron oxide. Alternatively, the iron oxide may be coated iron oxide particles in which iron oxide is coated with a surface treatment agent. The surface treatment of the iron oxide can be carried out primarily for the purpose of improving the uniformity of application state of the cosmetic and the stability of the formulation. The coated iron oxide may be iron oxide coated with a hydrophobic treatment agent, similarly to the metal oxide fine particles of the component (A). The iron oxide that is either uncoated or coated with a surface treatment agent, having an average minor axis length of, for example, 1000 nm or less and greater than 60 nm can be used, but as long as it functions as the coloring component, it is not limited to one having any particular minor axis length. When the component (C) is contained, the content may be, for example, 13% by mass or less based on the total mass of the cosmetic taken as 100%. When the iron oxide is contained in an amount of more than 13% by mass, the stability may be reduced, and the color may be too intense, which may impair the makeup quality. The content of the iron oxide may be, for example, 0.3 to 10% by mass, and is preferably 0.5 to 6% by mass. It should be noted that the iron oxide is an optional component, and when the appearance or application state of the cosmetic, in a lotion, an emulsion, a cream, a makeup base, or a lipstick, would be aesthetically impaired, the iron oxide may not be contained.

### (D) Volatile oil

A volatile oil is a liquid component that, in a cosmetic, facilitates spreading in application and, since at least a portion thereof evaporates after application, enables finish with reduced oil-induced gloss. As the volatile oil, a volatile noncyclic silicone oil, a volatile hydrocarbon oil, a volatile ester oil, or a combination thereof may be used. Volatile cyclic silicone oils are not included in the volatile oils used as the component (D). As a specific compound, one or more selected from dimethyl polysiloxane, methyl trimethicone, isododecane, undecane, tridecane coconut oil methyl esters, coconut oil ethyl esters, palm oil methyl esters, palm oil ethyl esters, palm oil butyl esters, butyl acetate, and ethyl acetate may be used, but the invention is not limited thereto. When the component (D) is contained, the content may be, for example, 60% by mass or less based on the total mass of the cosmetic taken as 100%. When the volatile oil is contained in an amount of more than 60% by mass, the stability of the cosmetic may be reduced in some cases. The content of the volatile oil may be, for example, 2 to 40% by mass, and is preferably 8 to 20% by mass. The volatile oil is an optional component. When the cosmetic can be sufficiently spread owing to the product form and the relationship with another component, when the cosmetic is less glossy due to a high powder content, when the cosmetic is solidified with a wax or the like and hence is less glossy, or when the cosmetic is not filled in a sealed container and the formulation stability will be lowered if a volatile component is blended, the desired properties can be achieved even without blending the volatile oil.

### (E) White pigment titanium dioxide

A white pigment titanium dioxide functions as a white colorant in a cosmetic. The white pigment titanium dioxide used as the component (E) may be a titanium dioxide having a minor axis D50 greater than that of the metal oxide fine particles used as the component (A), and a minor axis D90/D10 of more than 2.1, and more specifically may be a titanium dioxide having a minor axis D50 of, for example, 200 nm or more, and preferably 200 to 300 nm. The white pigment titanium dioxide may also be a titanium dioxide having a coated surface, similarly to the metal oxide fine particles of the component (A), and may be a titanium dioxide having a surface coated with a hydrophobic treatment agent, and the shape is not particularly limited and may be, for example, spherical, spindle-shaped, granular, or needle-like.

When the component (E) is contained, the content may be, for example, 11% by mass or less, and is preferably 5% by mass or less based on the total mass of the cosmetic taken as 100%. When the white pigment titanium dioxide is contained in an amount of more than 11% by mass, the cosmetic may be excessively white, which impairs the makeup quality, and may sink into the unevenness of the skin, resulting in a non-uniform finish. The content of the white pigment titanium dioxide may be further preferably 0.3 to 5% by mass, and may be 0.5 to 2% by mass. In addition, the content of the white pigment titanium dioxide of the component (E) is preferably determined such that the total amount of the components (A) and (E) is 30% by mass or less based on the total mass of the cosmetic taken as 100%, and more preferably determined such that the total amount of the components (A) and (E) is 25% by mass or less. When the total amount of the components (A) and (E) is more than 30% by mass, safety concerns may arise regardless of the particle size. In addition, the applied cosmetic film may be excessively thick, resulting in an unnatural appearance in some cases. It may also be preferable in some cases for the mass ratio of the components (A) and (E) to be (A):(E) = 50:1 to 1.5:1. The white pigment titanium dioxide is an optional component. When the appearance or application state of the cosmetic, in a lotion, an emulsion, a cream, a makeup base, or a lipstick, is aesthetically impaired, or when the component (A) is contained in an amount of 30% by mass, the white pigment titanium dioxide may not be contained in some cases. Mica coated with titanium dioxide that is used as a pearl pigment does not fall under the definition of the white pigment titanium dioxide.

Examples of other optional components include powders that do not fall under the definitions of the components (A), (B), (C), and (E). Such powders can be appropriately blended in the cosmetic for purposes of, for example, imparting color, providing makeup effects, adjusting texture, and the like. Such powders are not particularly limited as long as they are powders commonly used in cosmetics, and examples include, but are not limited to, talc, sericite, mica, kaolin, anhydrous silica, barium sulfate, and inorganic powders having an average minor axis length of 200 nm or more and less than about 1000 nm, such as zinc oxide, cerium oxide, magnesium oxide, aluminum oxide, and boron nitride, polymer powders such as silk and cellulose, an organic pigment, and composite powders thereof. In an embodiment, amorphous silica, either having a coated surface or an uncoated surface, may be added for purposes of, for example, lightening the spreadability, eliminating tackiness, scattering light to achieve a blurring effect, or enhancing ultraviolet protection. The amorphous silica preferably has oil absorption of 150 ml/100 g or less, and more preferably 70 ml/100 g or less. These optional powders may be surface treated powders. It is also preferable that none of these optional powders contain particles having a minor axis length of 60 nm or less.

Other examples of the optional components include those commonly used in cosmetics, quasi-drugs, and pharmaceutical compositions. Examples include an oil, a dye, a polymer compound, a fragrance, a surfactant, an antioxidant, a preservative, a humectant, a polyhydric alcohol, a lower alcohol, a saccharide, an ultraviolet absorber, a whitening agent, medical agents such as a skin-activating agent, a blood-circulation promoter, an anti-seborrheic agent, and an anti-inflammatory agent, an astringent, and a cooling agent. As an optional liquid component, for example, a nonvolatile liquid oil may be contained. Such a liquid oil may be contained as a base material, or for the purpose of imparting emollient properties. The liquid oil may be a nonvolatile oil that is liquid at 25°C, and is not particularly limited, and may be, for example, nonvolatile hydrocarbon oils such as liquid paraffin, squalane, and olefin oligomers (excluding volatile ones); ester oils such as 2-ethylhexanoic acid triglyceride, cetyl 2-ethylhexanoate, pentaerythrityl 2-ethylhexanoate, trimethylolpropane 2-ethylhexanoate, 2-ethylhexyl palmitate, isocetyl isononanoate, isopropyl myristate, cetyl 2-ethylhexanoate, diethyl sebacate, and diethyl adipate; and natural vegetable oils such as jojoba oil, olive oil, macadamia nut oil, cottonseed oil, tea seed oil, safflower oil, and rice bran oil.

Next, a method for producing the cosmetic of the present invention varies depending on the dosage form, and the cosmetic can be prepared by mixing the components according to a general production method for each dosage form, as described in detail in Examples.

Specific examples of the oily or emulsified makeup cosmetic of the present invention are not particularly limited, but include product forms such as a lotion containing a powder, an emulsion, a cream, a foundation, a primer, a makeup base, a concealer, a blush, an eye shadow, an eyeliner, a mascara, an eyebrow, a lipstick, and a nail enamel. Examples of the foundation include a water-in-oil type emulsified liquid foundation, an oil-in-water type emulsified liquid foundation, a water-in-oil type emulsified cream foundation, an oil-in-water type emulsified cream foundation, an oily cream foundation, a water-in-oil type emulsified stick foundation, and an oily stick foundation. Examples of the makeup base include a water-in-oil type emulsified cream base, and an oil-in-water type emulsified cream base. Examples of the concealer include an oily solid concealer, and examples of the cream include a lip cream, a sunscreen cream, a moisturizing cream, a whitening cream, and a beauty cream. These products may also have a function as a sunscreen cosmetic.

### EXAMPLES

Now, the present invention will be described in more detail with reference to Examples. The present invention, however, is not limited to Examples described below. Based on the compositions of Examples and Comparative Examples shown in tables below, cosmetics (water-in-oil type emulsified foundations) were prepared and evaluated for their properties.

### 1. Metal oxide fine particles

Metal oxide fine particles satisfying the condition for the component A, that is, titanium dioxide (D50 = 109 nm) (A1), titanium dioxide (D50 = 112 nm) (A2), and titanium dioxide (D50 = 128 nm) (A3) (all of which were spindle-shaped), and these titanium dioxides surface-coated with a hydrophobic treatment agent, were purchased from Titan Kogyo, Ltd. Similarly, zinc oxide (D50 = 110 nm, D90/D10 = 1.93, granular-shaped) (A4) and the zinc oxide surface-coated with a hydrophobic treatment agent were provided by TAYCA CORPORATION.

### 2. Preparation of water-in-oil type emulsified foundation

Water-in-oil type emulsified foundations were prepared by mixing, with a disperser, oily components that optionally contained the component D out of components shown in Tables 1 to 5, adding and dispersing a powder containing the component A, the component B, and optionally the component C prepared by the methods described above therein, and then, adding an aqueous phase, which was separately prepared by dissolving a water-soluble component in water, to the resulting mixture, and emulsifying the resultant by a stirring treatment.

### 3. Evaluation

Evaluation methods and criteria for each cosmetic were as follows.

### <Spreadability, finish uniformity, and matte texture on the skin (lack of gloss)>

### [Evaluation methods]

Each sample of Examples and Comparative Examples was actually used by a panel of ten experts for evaluation on three criteria, spreadability, finish uniformity, and matte texture on the skin (lack of gloss). Spreadability refers to how easily the cosmetic could be spread on the skin with low resistance after being taken on a finger, and a lower resistance was rated as a higher evaluation. Finish uniformity refers to a state in which the powder component of the cosmetic was applied to the skin surface in a uniform thickness, and a higher uniformity in the color and thickness of the cosmetic film without settling into the texture was rated as a higher evaluation. Matte texture on the skin (lack of gloss) refers to a skin surface condition after applying the cosmetic, in which light was diffusely reflected to reduce glossiness, and a case in which the skin was not glossy and looks to have a finer texture was rated as a higher evaluation. For all the items, an evaluation of A was assigned to samples rated as high by 8 or more out of the 10 panelists; an evaluation of B was assigned to samples rated as high by 5 to 7 out of the 10 panelists; and an evaluation of C was assigned to samples rated as high by 3 to 4 out of the 10 panelists. An evaluation of D was assigned to samples rated as high by 2 or fewer out of the 10 panelists.

### <Stability over time at 40°C>

### [Evaluation method]

The obtained cosmetics were allowed to stand at 40°C, and the conditions after 4, 6, and 8 weeks were visually observed and evaluated according to the following criteria.

### [Evaluation criteria]

A: No separation was observed after 8 weeks.
B: No separation was observed after 6 weeks, but a partial separation of oil was observed after 8 weeks.
C: No separation was observed after 4 weeks, but a partial separation of oil was observed after 6 weeks.
D: A partial separation of oil was observed after 4 weeks.

### <In vitro SPF>

### [Evaluation method]

Each sample was dropped in an amount of 2 mg/cm² onto a PMMA plate (Helioplate HD6, manufactured by Helioscreen) to be applied using a finger for 60 seconds, and the resultant was dried for 15 minutes to form a cosmetic film. Using, as a control, a plate on which no sample was applied, the absorbance (290 to 450 nm) of the cosmetic film was measured with a UV2000S analyzer manufactured by Labsphere, and based on the thus obtained measurement data, an *in vitro* SPF (Sun Protection Factor) value was calculated.

### 4. Results

The compositions and evaluation results of Examples and Comparative Examples are shown in Tables 1 to 5 below. Numerical values regarding the formulations listed in the tables all represent "percentages by mass" based on the total mass of the cosmetic taken as 100%. A symbol "-" indicates that the corresponding component was not contained. In Tables 1 and 2, each value in parentheses following the name of titanium dioxide is the D50 value. In Examples and Comparative Examples below, *1 indicates that the minor axis D50 = 109 nm, and D90/D10 = 1.25 in the corresponding particles, *2 indicates that the minor axis D50 = 270 nm, D90/D10 = 4.20, D10 = 156, and D90 = 655 in the titanium dioxide particles, and *3 indicates that the minor axis D50 = 110 nm, and D90/D10 = 1.93 in the zinc oxide particles A4.

**[Table 1]**

| | Name | D50 | D90 /D10 | Example | | | Comparative Example | | | |
|---|---|---|---|---|---|---|---|---|---|---|
| | | | | 1 | 2 | 3 | 1 | 2 | 3 | 4 |
| A | Stearic acid/aluminum hydroxide-coated titanium dioxide A1 (109 nm) | 109 | 1.25 | 16 | - | - | - | - | - | - |
| | Stearic acid/aluminum hydroxide-coated titanium dioxide A2 (112 nm) | 112 | 1.28 | - | 16 | - | - | - | - | - |
| | Stearic acid/aluminum hydroxide-coated titanium dioxide A3 (128 nm) | 128 | 1.62 | - | - | 16 | - | - | - | - |
| | Stearic acid/aluminum hydroxide-coated titanium dioxide (30 nm) | 30 | 1.78 | - | - | - | 16 | - | - | - |
| | Stearic acid/aluminum hydroxide-coated titanium dioxide (160 nm) | 160 | 1.82 | - | - | - | - | 16 | - | - |
| | Stearic acid/aluminum hydroxide-coated titanium dioxide (28 nm) | 28 | 2.82 | - | - | - | - | - | 16 | - |
| | Stearic acid/aluminum hydroxide-coated titanium dioxide (97 nm) | 97 | 4.60 | - | - | - | - | - | - | 16 |
| | Stearic acid/aluminum hydroxide-coated titanium dioxide (179 nm) | 179 | 3.12 | - | - | - | - | - | - | - |
| B | Disteardimonium hectorite | - | - | 1 | 1 | 1 | 1 | 1 | 1 | 1 |
| E | White pigment titanium dioxide *2 | 270 | 4.20 | 0.5 | 0.5 | 0.5 | 0.5 | 0.5 | 0.5 | 0.5 |
| | Mica | - | - | 8.44 | 8.44 | 8.44 | 8.44 | 8.44 | 8.44 | 8.44 |
| C | Yellow iron oxide | - | - | 0.5 | 0.5 | 0.5 | 0.5 | 0.5 | 0.5 | 0.5 |
| | Red iron oxide | - | - | 0.05 | 0.05 | 0.05 | 0.05 | 0.05 | 0.05 | 0.05 |
| | Black iron oxide | - | - | 0.01 | 0.01 | 0.01 | 0.01 | 0.01 | 0.01 | 0.01 |
| D | Methyl trimethicone (volatile) | - | - | 4.9 | 4.9 | 4.9 | 4.9 | 4.9 | 4.9 | 4.9 |
| | Dimethyl polysiloxane (volatile) | - | - | 8 | 8 | 8 | 8 | 8 | 8 | 8 |
| | Caprylic/capric triglyceride | - | - | 2 | 2 | 2 | 2 | 2 | 2 | 2 |
| | Neopentyl glycol diethylhexanoate | - | - | 4 | 4 | 4 | 4 | 4 | 4 | 4 |
| | PEG-10Dimethicone | - | - | 3.5 | 3.5 | 3.5 | 3.5 | 3.5 | 3.5 | 3.5 |
| | (Dimethicone/vinyl dimethicone) crosspolymer | | | 0.5 | 0.5 | 0.5 | 0.5 | 0.5 | 0.5 | 0.5 |
| | Magnesium stearate-coated amorphous silica (oil absorption: 60 mL/100 g) | | | 3 | 3 | 3 | 3 | 3 | 3 | 3 |
| | Water | - | - | balance | balance | balance | balance | balance | balance | balance |
| | DPG | - | - | 5 | 5 | 5 | 5 | 5 | 5 | 5 |
| | Pentylene glycol | - | - | 2 | 2 | 2 | 2 | 2 | 2 | 2 |
| | BG | - | - | 1.5 | 1.5 | 1.5 | 1.5 | 1.5 | 1.5 | 1.5 |
| | Glycerin | - | - | 1 | 1 | 1 | 1 | 1 | 1 | 1 |
| | Phenoxyethanol | - | - | 0.7 | 0.7 | 0.7 | 0.7 | 0.7 | 0.7 | 0.7 |
| | Polyglyceryl-2 diisostearate | - | - | 1 | 1 | 1 | 1 | 1 | 1 | 1 |
| | Sorbitol | - | - | 0.9 | 0.9 | 0.9 | 0.9 | 0.9 | 0.9 | 0.9 |
| | Citric acid | - | - | 0.1 | 0.1 | 0.1 | 0.1 | 0.1 | 0.1 | 0.1 |
| | Sodium citrate | - | - | 0.5 | 0.5 | 0.5 | 0.5 | 0.5 | 0.5 | 0.5 |
| | Trimethylsiloxysilicate | - | - | 0.5 | 0.5 | 0.5 | 0.5 | 0.5 | 0.5 | 0.5 |
| | Sodium chloride | - | - | 1 | 1 | 1 | 1 | 1 | 1 | 1 |
| | Magnesium sulfate | - | - | 1 | 1 | 1 | 1 | 1 | 1 | 1 |
| | Tocopherol | - | - | 0.2 | 0.2 | 0.2 | 0.2 | 0.2 | 0.2 | 0.2 |
| | Monosodium glutamate | - | - | 1 | 1 | 1 | 1 | 1 | 1 | 1 |
| | Sodium hyaluronate | - | - | 0.1 | 0.1 | 0.1 | 0.1 | 0.1 | 0.1 | 0.1 |
| Evaluation | Stability over time at 40°C | - | - | A | A | B | A | C | A | A |
| | Spreadability | - | - | A | A | A | C | C | D | C |
| | Finish uniformity | - | - | A | A | B | C | B | C | D |
| | Matte texture on skin (lack of gloss) | - | - | A | A | A | D | B | D | C |
| | in vitro SPF | - | - | 51 | 46 | 40 | 66 | 17 | 68 | 39 |

**[Table 2]**

| | Name | D50 | D90 /D10 | Comparative Example | Example | Comparative Example | Example | | | Comparative Example |
|---|---|---|---|---|---|---|---|---|---|---|
| | | | | 5 | 4 | 6 | 5 | 6 | 7 | 7 |
| A | Stearic acid/aluminum hydroxide-coated titanium dioxide A1 (109 nm) | 109 | 1.25 | - | 11 | - | 2 | 6 | 19 | 26 |
| | Stearic acid/aluminum hydroxide-coated titanium dioxide (28 nm) | 28 | 2.82 | - | 4.5 | 5 | - | - | - | - |
| | Stearic acid/aluminum hydroxide-coated titanium dioxide (179 nm) | 179 | 3.12 | 16 | - | - | - | - | - | - |
| B | Disteardimonium hectorite | - | - | 1 | 1 | 1 | 1 | 1 | 1 | 1 |
| E | White pigment titanium dioxide *2 | 270 | 4.20 | 0.5 | 0.5 | 11.5 | 0.5 | 0.5 | 0.5 | 0.5 |
| | Mica | - | - | 8.44 | 8.44 | 8.44 | 8.44 | 8.44 | 8.44 | 8.44 |
| C | Yellow iron oxide | - | - | 0.5 | 0.5 | 0.5 | 0.5 | 0.5 | 0.5 | 0.5 |
| | Red iron oxide | - | - | 0.05 | 0.05 | 0.05 | 0.05 | 0.05 | 0.05 | 0.05 |
| | Black iron oxide | - | - | 0.01 | 0.01 | 0.01 | 0.01 | 0.01 | 0.01 | 0.01 |
| D | Methyl trimethicone (volatile) | - | - | 4.9 | 4.9 | 4.9 | 4.9 | 4.9 | 4.9 | 4.9 |
| | Dimethyl polysiloxane (volatile) | - | - | 8 | 8 | 8 | 8 | 8 | 8 | 8 |
| | Caprylic/capric triglyceride | - | - | 2 | 2 | 2 | 2 | 2 | 2 | 2 |
| | Neopentyl glycol diethylhexanoate | - | - | 4 | 4 | 4 | 4 | 4 | 4 | 4 |
| | PEG-10 Dimethicone | - | - | 3.5 | 3.5 | 3.5 | 3.5 | 3.5 | 3.5 | 3.5 |
| | (Dimethicone/vinyl dimethicone) crosspolymer | - | - | 0.5 | 0.5 | 0.5 | 0.5 | 0.5 | 0.5 | 0.5 |
| | Magnesium stearate-coated amorphous silica (oil absorption: 60 mL/100 g) | - | - | 3 | 3 | 3 | 3 | 3 | 3 | 3 |
| | Water | - | - | balance | balance | balance | balance | balance | balance | balance |
| | DPG | - | - | 5 | 5 | 5 | 5 | 5 | 5 | 5 |
| | Pentylene glycol | - | - | 2 | 2 | 2 | 2 | 2 | 2 | 2 |
| | BG | - | - | 1.5 | 1.5 | 1.5 | 1.5 | 1.5 | 1.5 | 1.5 |
| | Glycerin | - | - | 1 | 1 | 1 | 1 | 1 | 1 | 1 |
| | Phenoxyethanol | - | - | 0.7 | 0.7 | 0.7 | 0.7 | 0.7 | 0.7 | 0.7 |
| | Polyglyceryl-2 diisostearate | - | - | 1 | 1 | 1 | 1 | 1 | 1 | 1 |
| | Sorbitol | - | - | 0.9 | 0.9 | 0.9 | 0.9 | 0.9 | 0.9 | 0.9 |
| | Citric acid | - | - | 0.1 | 0.1 | 0.1 | 0.1 | 0.1 | 0.1 | 0.1 |
| | Sodium citrate | - | - | 0.5 | 0.5 | 0.5 | 0.5 | 0.5 | 0.5 | 0.5 |
| | Trimethylsiloxysilicate | - | - | 0.5 | 0.5 | 0.5 | 0.5 | 0.5 | 0.5 | 0.5 |
| | Sodium chloride | - | - | 1 | 1 | 1 | 1 | 1 | 1 | 1 |
| | Magnesium sulfate | - | - | 1 | 1 | 1 | 1 | 1 | 1 | 1 |
| | Tocopherol | - | - | 0.2 | 0.2 | 0.2 | 0.2 | 0.2 | 0.2 | 0.2 |
| | Monosodium glutamate | - | - | 1 | 1 | 1 | 1 | 1 | 1 | 1 |
| | Sodium hyaluronate | - | - | 0.1 | 0.1 | 0.1 | 0.1 | 0.1 | 0.1 | 0.1 |
| Evaluation | Stability over time at 40°C | - | - | B | A | B | A | A | B | C |
| | Spreadability | - | - | B | C | B | A | A | C | C |
| | Finish uniformity | - | - | D | B | D | B | B | A | B |
| | Matte texture on skin (lack of gloss) | - | - | C | B | C | C | B | A | A |
| | in vitro SPF | - | - | 23 | 60 | 25 | 5 | 22 | 55 | 79 |

**[Table 3]**

| | Name | Example 1 | Comparative Example 8 | Comparative Example 9 | Example 8 | Example 9 | Example 10 |
|---|---|---|---|---|---|---|---|
| A | Stearic acid/aluminum hydroxide-coated titanium dioxide A1 *1 | 16 | 16 | 16 | 16 | 16 | 16 |
| B | Disteardimonium hectorite | 1 | - | 0.2 | 0.4 | - | - |
| | Bentonite | - | - | - | - | 1 | - |
| | Quaternium-90 bentonite | - | - | - | - | - | 1 |
| E | White pigment titanium dioxide *2 | 0.5 | 0.5 | 0.5 | 0.5 | 0.5 | 0.5 |
| | Mica | 8.44 | 8.44 | 8.44 | 8.44 | 8.44 | 8.44 |
| C | Yellow iron oxide | 0.5 | 0.5 | 0.5 | 0.5 | 0.5 | 0.5 |
| | Red iron oxide | 0.05 | 0.05 | 0.05 | 0.05 | 0.05 | 0.05 |
| | Black iron oxide | 0.01 | 0.01 | 0.01 | 0.01 | 0.01 | 0.01 |
| D | Methyl trimethicone (volatile) | 4.9 | 4.9 | 4.9 | 4.9 | 4.9 | 4.9 |
| | Dimethyl polysiloxane (volatile) | 8 | 8 | 8 | 8 | 8 | 8 |
| | Caprylic/capric triglyceride | 2 | 2 | 2 | 2 | 2 | 2 |
| | Neopentyl glycol diethylhexanoate | 4 | 4 | 4 | 4 | 4 | 4 |
| | PEG-10 Dimethicone | 3.5 | 3.5 | 3.5 | 3.5 | 3.5 | 3.5 |
| | (Dimethicone/vinyl dimethicone) crosspolymer | 0.5 | 0.5 | 0.5 | 0.5 | 0.5 | 0.5 |
| | Magnesium stearate-coated amorphous silica (oil absorption: 60 mL/100 g) | 3 | 3 | 3 | 3 | 3 | 3 |
| | Water | balance | balance | balance | balance | balance | balance |
| | DPG | 5 | 5 | 5 | 5 | 5 | 5 |
| | Pentylene glycol | 2 | 2 | 2 | 2 | 2 | 2 |
| | BG | 1.5 | 1.5 | 1.5 | 1.5 | 1.5 | 1.5 |
| | Glycerin | 1 | 1 | 1 | 1 | 1 | 1 |
| | Phenoxyethanol | 0.7 | 0.7 | 0.7 | 0.7 | 0.7 | 0.7 |
| | Polyglyceryl-2 diisostearate | 1 | 1 | 1 | 1 | 1 | 1 |
| | Sorbitol | 0.9 | 0.9 | 0.9 | 0.9 | 0.9 | 0.9 |
| | Citric acid | 0.1 | 0.1 | 0.1 | 0.1 | 0.1 | 0.1 |
| | Sodium citrate | 0.5 | 0.5 | 0.5 | 0.5 | 0.5 | 0.5 |
| | Trimethylsiloxysilicate | 0.5 | 0.5 | 0.5 | 0.5 | 0.5 | 0.5 |
| | Sodium chloride | 1 | 1 | 1 | 1 | 1 | 1 |
| | Magnesium sulfate | 1 | 1 | 1 | 1 | 1 | 1 |
| | Tocopherol | 0.2 | 0.2 | 0.2 | 0.2 | 0.2 | 0.2 |
| | Monosodium glutamate | 1 | 1 | 1 | 1 | 1 | 1 |
| | Sodium hyaluronate | 0.1 | 0.1 | 0.1 | 0.1 | 0.1 | 0.1 |
| Evaluation | Stability over time at 40°C | A | D | C | B | B | A |
| | Spreadability | A | A | A | A | A | A |
| | Finish uniformity | A | D | C | B | B | A |
| | Matte texture on skin (lack of gloss) | A | B | B | A | A | A |
| | in vitro SPF | 51 | 40 | 44 | 45 | 39 | 53 |

**[Table 4]**

| | Name | Example 11 | Example 12 | Example 13 | Example 14 | Example 15 |
|---|---|---|---|---|---|---|
| A | Stearic acid/aluminum hydroxide-coated titanium dioxide A1 *1 | 16 | 16 | 16 | 16 | 16 |
| B | Disteardimonium hectorite | 4 | 1 | 1 | 1 | 1 |
| E | White pigment titanium dioxide *2 | 0.5 | 0.5 | 0.5 | - | 10 |
| | Mica | 8.44 | 8.44 | 8.44 | 8.44 | 8.44 |
| C | Yellow iron oxide | 0.5 | - | 10 | 0.5 | 0.5 |
| | Red iron oxide | 0.05 | - | 1 | 0.05 | 0.05 |
| | Black iron oxide | 0.01 | - | 0.2 | 0.01 | 0.01 |
| D | Methyl trimethicone (volatile) | 4.9 | 4.9 | 4.9 | 4.9 | 4.9 |
| | Dimethyl polysiloxane (volatile) | 8 | 8 | 8 | 8 | 8 |
| | Caprylic/capric triglyceride | 2 | 2 | 2 | 2 | 2 |
| | Neopentyl glycol diethylhexanoate | 4 | 4 | 4 | 4 | 4 |
| | PEG-10 Dimethicone | 3.5 | 3.5 | 3.5 | 3.5 | 3.5 |
| | (Dimethicone/vinyl dimethicone) crosspolymer | 0.5 | 0.5 | 0.5 | 0.5 | 0.5 |
| | Magnesium stearate-coated amorphous silica (oil absorption: 60 mL/100 g) | 3 | 3 | 3 | 3 | 3 |
| | Water | balance | balance | balance | balance | balance |
| | DPG | 5 | 5 | 5 | 5 | 5 |
| | Pentylene glycol | 2 | 2 | 2 | 2 | 2 |
| | BG | 1.5 | 1.5 | 1.5 | 1.5 | 1.5 |
| | Glycerin | 1 | 1 | 1 | 1 | 1 |
| | Phenoxyethanol | 0.7 | 0.7 | 0.7 | 0.7 | 0.7 |
| | Polyglyceryl-2 diisostearate | 1 | 1 | 1 | 1 | 1 |
| | Sorbitol | 0.9 | 0.9 | 0.9 | 0.9 | 0.9 |
| | Citric acid | 0.1 | 0.1 | 0.1 | 0.1 | 0.1 |
| | Sodium citrate | 0.5 | 0.5 | 0.5 | 0.5 | 0.5 |
| | Trimethylsiloxysilicate | 0.5 | 0.5 | 0.5 | 0.5 | 0.5 |
| | Sodium chloride | 1 | 1 | 1 | 1 | 1 |
| | Magnesium sulfate | 1 | 1 | 1 | 1 | 1 |
| | Tocopherol | 0.2 | 0.2 | 0.2 | 0.2 | 0.2 |
| | Monosodium glutamate | 1 | 1 | 1 | 1 | 1 |
| | Sodium hyaluronate | 0.1 | 0.1 | 0.1 | 0.1 | 0.1 |
| Evaluation | Stability over time at 40°C | A | A | C | B | B |
| | Spreadability | C | A | B | A | C |
| | Finish uniformity | A | C | C | B | C |
| | Matte texture on skin (lack of gloss) | C | A | A | B | B |
| | in vitro SPF | 57 | 50 | 55 | 44 | 55 |

**[Table 5]**

| | Name | Example 1 | Comparative Example 10 | Example 16 | Example 17 |
|---|---|---|---|---|---|
| A | Stearic acid/aluminum hydroxide-coated titanium dioxide A1 *1 | 16 | 16 | 4 | 16 |
| | Dimethicone-coated zinc oxide A4 *3 | - | - | 14 | - |
| B | Disteardimonium hectorite | 1 | 1 | 1 | 1 |
| E | White pigment titanium dioxide *2 | 0.5 | 0.5 | 0.5 | 0.5 |
| | Mica | 8.44 | 8.44 | 8.44 | 8.44 |
| C | Yellow iron oxide | 0.5 | 0.5 | 0.5 | 0.5 |
| | Red iron oxide | 0.05 | 0.05 | 0.05 | 0.05 |
| | Black iron oxide | 0.01 | 0.01 | 0.01 | 0.01 |
| D | Methyl trimethicone (volatile) | 4.9 | 4.9 | 4.9 | - |
| | Dimethyl polysiloxane (volatile) | 8 | - | 8 | - |
| volatile cyclic silicone | Decamethylcyclopentasiloxane | - | 12.9 | - | - |
| | Dimethyl polysiloxane(nonvolatile) | - | - | - | 12.9 |
| MPB | Polymethylsilsesquioxane | - | 1 | - | - |
| | Caprylic/capric triglyceride | 2 | 2 | 2 | 2 |
| | Neopentyl glycol diethylhexanoate | 4 | 4 | 4 | 4 |
| | PEG-10 Dimethicone | 3.5 | 3.5 | 3.5 | 3.5 |
| | (Dimethicone/vinyl dimethicone) crosspolymer | 0.5 | 0.5 | 0.5 | 0.5 |
| | Magnesium stearate-coated amorphous silica (oil absorption: 60 mL/100 g) | 3 | 3 | 3 | 3 |
| | Water | balance | balance | balance | balance |
| | DPG | 5 | 5 | 5 | 5 |
| | Pentylene glycol | 2 | 2 | 2 | 2 |
| | BG | 1.5 | 1.5 | 1.5 | 1.5 |
| | Glycerin | 1 | 1 | 1 | 1 |
| | Phenoxyethanol | 0.7 | 0.7 | 0.7 | 0.7 |
| | Polyglyceryl-2 diisostearate | 1 | 1 | 1 | 1 |
| | Sorbitol | 0.9 | 0.9 | 0.9 | 0.9 |
| | Citric acid | 0.1 | 0.1 | 0.1 | 0.1 |
| | Sodium citrate | 0.5 | 0.5 | 0.5 | 0.5 |
| | Trimethylsiloxysilicate | 0.5 | 0.5 | 0.5 | 0.5 |
| | Sodium chloride | 1 | 1 | 1 | 1 |
| | Magnesium sulfate | 1 | 1 | 1 | 1 |
| | Tocopherol | 0.2 | 0.2 | 0.2 | 0.2 |
| | Monosodium glutamate | 1 | 1 | 1 | 1 |
| | Sodium hyaluronate | 0.1 | 0.1 | 0.1 | 0.1 |
| Evaluation | Stability over time at 40°C | A | A | A | A |
| | Spreadability | A | A | A | A |
| | Finish uniformity | A | A | A | B |
| | Matte texture on skin (lack of gloss) | A | A | A | B |
| | in vitro SPF | 51 | 45 | 40 | 29 |

As shown in Tables 1 to 5, the cosmetics of Examples according to the present invention, owing to the compositions that were free of microplastic beads and volatile cyclic silicones, and were minimized in the content of metal oxide particles with a minor axis length of 60 nm or less, were able to achieve the uniform finish, good spreadability, and lack of gloss like conventional compositions containing microplastic beads and volatile cyclic silicones. Example 4 contained titanium dioxide particles having D50 = 28 nm, which was smaller than that of the component A, but considering the presence of a particle size distribution, the content of particles having a minor axis length of 60 nm or less was less than 5% by mass. Furthermore, the other Examples did not contain particles with a minor axis length smaller than that of the component A, and therefore, particles with a minor axis length of 60 nm or less were substantially not contained, or were contained in an amount of less than 1%. In addition, cosmetics capable of achieving particularly high SPF values through ultraviolet scattering effects without using UV absorbers were also obtained.

### 5. Formulation examples

Formulation examples of the cosmetic of the present invention will be described below. The methods for preparing the cosmetics for the respective formulation examples were as follows:

### <Preparation methods>

### (Formulation Examples 1, 3, 5, 6, and 13)

Each cosmetic having the composition listed as the example below was prepared by dissolving and mixing an aqueous component in water with a disper, heating the resultant mixture to 70°C, adding thereto a separately prepared mixture heated to 80°C in which an oily component and a powder were mixed and dispersed, and emulsifying the resultant mixture by a stirring treatment.

### (Formulation Examples 2 and 4)

Each cosmetic having the composition listed as the example below was prepared by adding, to a mixture obtained by mixing an oily component with a disper and further adding and dispersing a powder therein, an aqueous phase component separately obtained by dissolving a water-soluble component in water, and emulsifying the resultant by a stirring treatment.

### (Preparation Example 7)

A cosmetic having the composition listed as the example below was prepared by adding, to a mixture obtained by adding and dispersing a powder to and in an oil phase having been dissolved by heating to 80°C or more, an aqueous phase component that had been separately obtained by dissolving and mixing an aqueous component in water, and had been heated to 80°C or more, and emulsifying the resultant by a stirring treatment.

### (Formulation Examples 8 to 12)

Each cosmetic having the composition listed as the example below was prepared by filling, in a container or a metal mold, a mixture obtained by mixing and dissolving an oily component with a disper by heating to 80°C or more, and dispersing a powder therein, and cooling the resultant.

None of the cosmetics of the following Formulation Examples 1 to 13 contained fine particles with a D50 smaller than that of the component A, and all of the cosmetics of these formulation examples were cosmetics that can solve the problems to be solved by the present invention without using conventionally employed components, and can achieve a uniform finish, excellent stability, and an *in vitro* SPF of 15 or higher.

### Formulation Example 1: Oil-in-water type emulsified liquid foundation

| | |
|---|---|
| Titanium dioxide A1 (component A) | 5.6 |
| (dimethicone-coated, D50 = 109 nm) | |
| Bentonite (component B) | 0.6 |
| Dimethicone-coated yellow iron oxide (component C) | 0.5 |
| Dimethicone-coated red iron oxide (component C) | 0.1 |
| Amorphous silica (oil absorption: 60 ml/100g) | 5 |
| Lauroyl lysine-coated saccharomyces culture | 0.9 |
| (C13-15) Alkane (volatile) (component D) | 3 |
| Alkyl benzoate (C12-15) | 5 |
| Dicaprylyl carbonate | 2 |
| Squalane | 1 |
| Polysilicone-15 | 7 |
| Ethylhexyl salicylate | 5 |
| Diethylamino hydroxybenzoyl hexyl benzoate | 3 |
| Bis-ethylhexyloxyphenol methoxyphenyl triazine | 3 |
| Behenyl alcohol | 2 |
| Stearyl alcohol | 0.7 |
| Stearic acid | 1.5 |
| Stearyl stearate | 1 |
| Glyceryl stearate | 1.5 |
| PEG-10 stearate | 0.8 |
| Glyceryl stearate (SE) | 0.2 |
| PEG-60 glyceryl isostearate | 0.7 |
| Water | balance |
| Pentylene glycol | 2 |
| Ethanol | 5 |
| BG (butylene glycol) | 1 |
| Phenylbenzimidazole sulfonic acid | 2.7 |
| Sodium hydroxide | 0.8 |
| Glycerin | 0.5 |
| Polyglyceryl-2 triisostearate | 0.5 |
| Sclerotium gum | 0.15 |
| Xanthan gum | 0.2 |
| Citric acid | 0.15 |
| Sodium citrate | 0.05 |

### Formulation Example 2: Water-in-oil type cream base

| | |
|---|---|
| Titanium dioxide A2 (component A) | 11 |
| (stearic acid/aluminum hydroxide-coated, D50 = 112 nm) | |
| Zinc oxide A4 (component A) | 18 |
| (triethoxycaprylylsilane-coated, D50 = 110 nm) | |
| Disteardimonium hectorite (component B) | 0.5 |
| Quaternium-18 hectorite (component B) | 0.4 |
| White pigment titanium dioxide (component E) | 1 |
| (stearic acid/aluminum hydroxide-coated, D50 = 250 nm, D90 = 390 nm, D10 = 160 nm) | |
| Aluminum starch octenylsuccinate | 4 |
| Lauroyl lysine-coated saccharomyces culture | 0.5 |
| Methyl trimethicone (volatile) | 17 |
| (C13-15) Alkane (volatile) (component D) | 3 |
| Dimethicone (nonvolatile) | 5 |
| Alkyl benzoate (C12-15) | 7 |
| Polysilicone-15 | 9 |
| Ethylhexyl salicylate | 4.5 |
| Dicaprylyl carbonate | 3 |
| Bis-ethylhexyloxyphenol methoxyphenyl triazine | 1 |
| Diethylamino hydroxybenzoyl hexyl benzoate | 4 |
| Lauryl PEG-9 polydimethylsiloxyethyl dimethicone | 2 |
| Tocopherol | 0.1 |
| PEG-9 polydimethylsiloxyethyl dimethicone | 0.2 |
| Diisostearoyl polyglyceryl-3 dimer dilinoleate | 0.5 |
| Polyhydroxystearic acid | 0.4 |
| Isostearic acid | 0.3 |
| Sorbitan sesquiisostearate | 0.5 |
| Trimethylsiloxysilicate | 1.5 |
| Water | balance |
| Ethanol | 0.5 |
| Glycerin | 0.5 |
| Magnesium sulfate | 0.1 |
| Sodium hyaluronate | 0.1 |

### Formulation Example 3: Oil-in-water type cream foundation

| | |
|---|---|
| Titanium dioxide A1 (component A) (dimethicone-coated, D50 = 109 nm) | 11 |
| Bentonite (component B) | 0.6 |
| Zinc oxide A4 (component A) (dimethicone-coated, D50 = 110 nm) | 10 |
| Dimethicone-coated yellow iron oxide (component C) | 0.8 |
| Dimethicone-coated red iron oxide (component C) | 0.15 |
| Dimethicone-coated black iron oxide (component C) | 0.07 |
| Mica | 4 |
| Isotridecyl isononanoate | 6 |
| Bis-ethylhexyloxyphenol methoxyphenyl triazine | 3 |
| Polyglyceryl-3 disiloxane dimethicone | 3 |
| Tocopherol | 0.15 |
| Sorbitan sesquiisostearate | 0.7 |
| Polyglyceryl-2 triisostearate | 2.8 |
| Water | balance |
| (Sodium acrylate/sodium acryloyldimethyl taurate) Copolymer | 0.39 |
| (PEG-240/decyltetradeceth-20/HDI) copolymer | 0.95 |
| BG | 3 |
| Glycerin | 4 |
| Phenoxyethanol | 0.5 |
| Polysorbate 80 | 0.21 |
| PEG-60 hydrogenated castor oil | 0.8 |
| Pentylene glycol | 0.19 |
| Sodium hyaluronate | 0.1 |

### Formulation Example 4: Water-in-oil type cream foundation

| | |
|---|---|
| Titanium dioxide A1 (component A) | 14 |
| (isostearic acid-coated, D50 = 109 nm) | |
| Disteardimonium hectorite (component B) | 0.5 |
| White pigment titanium dioxide (component E) (stearic acid/aluminum hydroxide-coated, D50 = 250 nm, D90 = 390 nm,D10 = 160 nm) | 2 |
| Dimethicone-coated yellow iron oxide (component C) | 2.5 |
| Dimethicone-coated red iron oxide (component C) | 0.55 |
| Dimethicone-coated black iron oxide (component C) | 0.1 |
| Dimethicone-coated mica | 4 |
| Mg stearate-coated amorphous silica (oil absorption: 60 mL/100 g) | 1.2 |
| Dimethicone (volatile) (component D) | 8 |
| Diphenylsiloxy phenyl trimethicone | 0.75 |
| Cetyl PEG/PPG-10/1 dimethicone | 4 |
| Trimethylsiloxysilicate | 4 |
| Diisostearyl malate | 3 |
| (Dimethicone/vinyl dimethicone) crosspolymer | 0.6 |
| Polyglyceryl-2 diisostearate | 1.5 |
| Neopentyl glycol diethylhexanoate | 1.5 |
| Water | balance |
| Betaine | 1 |
| Glycerin | 3.49 |
| BG | 4 |
| Pentylene glycol | 1.5 |
| Diglycerin | 1 |
| Sorbitol | 1 |
| Magnesium sulfate | 1 |
| Phenoxyethanol | 0.3 |
| Sodium hyaluronate | 0.01 |

### Formulation Example 5: Oil-in-water type cream base

| | |
|---|---|
| Titanium dioxide A1 (component A) (aluminum hydroxide-coated, D50 = 109 nm) | 6 |
| (Al/Mg) silicate (component B) | 0.4 |
| Triethoxycaprylylsilane-coated yellow iron oxide (component C) | 0.06 |
| Triethoxycaprylylsilane-coated red iron oxide (component C) | 0.02 |
| Triethoxycaprylylsilane-coated black iron oxide (component C) | 0.01 |
| Iron oxide-coated titanium mica | 1 |
| Polyacrylate crosspolymer 6 | 0.7 |
| Cellulose acetate | 4 |
| Methyl trimethicone (volatile) (component D) | 2.5 |
| Polyglyceryl-2 triisostearate | 3 |
| Dicaprylyl carbonate | 3 |
| Squalane | 1 |
| Sorbitan isostearate | 0.2 |
| Hydrogenated lecithin | 0.5 |
| Ceramide | 0.2 |
| Hydrogenated polydecene | 9.5 |
| Water | balance |
| (Sodium acrylate/sodium acryloyldimethyl taurate) copolymer | 1.1 |
| Ethyl hexyl glycerin | 0.1 |
| DPG | 4.5 |
| Glycerin | 5 |
| Isopentyl diol | 1 |
| Pentylene glycol | 1 |
| Polysorbate 80 | 1.2 |
| Sodium citrate | 0.2 |
| Citric acid | 0.03 |

### Formulation Example 6: Oil-in-water type cream foundation

| | |
|---|---|
| Titanium dioxide A2 (component A) | 13 |
| (aluminum hydroxide-coated, D50 = 112 nm) | |
| Bentonite (component B) | 2 |
| White pigment titanium dioxide (component E) (D50 = 250 nm, D90 = 390 nm, D10 = 160 nm) | 1.4 |
| Silica-coated yellow iron oxide (component C) | 1 |
| Silica-coated red iron oxide (component C) | 0.3 |
| Silica-coated black iron oxide (component C) | 0.1 |
| Titanium dioxide coated mica(red) | 1.1 |
| Mica | 2 |
| Alumina | 0.5 |
| Amorphous silica (oil absorption: 60 ml/100 g) | 2 |
| (C9-12) Alkane (volatile) (component D) | 4 |
| Glyceryl stearate | 0.7 |
| Isostearic acid | 0.3 |
| Stearic acid | 1.4 |
| Behenyl alcohol | 1.5 |
| Hydrogenated coco-glycerides | 2 |
| Sucrose distearate | 0.3 |
| Sucrose pentaerucate | 0.5 |
| Polyglyceryl-10 laurate | 2.5 |
| Polyglyceryl-2 diisostearate | 1.2 |
| Squalane | 5 |
| Water | balance |
| BG | 3 |
| Propanediol | 4.4 |
| Glycerin | 5 |
| Phenoxyethanol | 0.5 |
| Diglycerin | 2.5 |
| Xanthan gum | 0.2 |
| Sodium citrate | 0.1 |
| Tocopherol | 0.05 |
| Sodium hydroxide | 0.1 |
| Glycosyl trehalose | 0.1 |
| Arginine | 0.2 |
| Sodium hyaluronate | 0.05 |

### Formulation Example 7: Water-in-oil type emulsified stick foundation

| | |
|---|---|
| Titanium dioxide A2 (component A) (dimethicone-coated, D50 = 120 nm) | 12 |
| Quaternium-18 hectorite (component B) | 0.5 |
| White pigment titanium dioxide (component E) (dimethicone/aluminum hydroxide-coated, D50 = 250 nm, D90 = 390 nm,D10 = 160 nm) | 3 |
| Dimethicone-coated yellow iron oxide (component C) | 1.4 |
| Dimethicone-coated red iron oxide (component C) | 0.3 |
| Dimethicone-coated black iron oxide (component C) | 0.1 |
| Dimethicone-coated mica | 3 |
| Dimethicone-coated amorphous silica (oil absorption: 60 ml/100 g) | 3 |
| Methyl trimethicone (volatile) (component D) | 8 |
| Polyoxyethylene methylpolysiloxane copolymer | 0.5 |
| Dimethicone (nonvolatile) | 9.5 |
| Crosslinked polyether-modified silicone | 4.5 |
| Glyceryl tri(2-ethylhexanoate) | 7.8 |
| Isotridecyl isononanoate | 2 |
| Dicaprylyl carbonate | 2 |
| Polysilicone-15 | 5 |
| Diethylamino hydroxybenzoyl hexyl benzoate | 2 |
| Ceramide | 0.1 |
| Natural vitamin E | 0.1 |
| Fragrance | 0.2 |
| Butylparaben | 0.1 |
| Paraffin wax | 3 |
| Candelilla Wax | 6.5 |
| Water | balance |
| Sodium citrate | 0.3 |
| 1,3-Butylene glycol | 4 |
| Glycerin | 1 |
| Hyaluronic acid | 0.1 |
| Sodium chloride | 0.4 |
| Phenoxyethanol | 0.1 |

### Formulation Example 8: Oily stick foundation

| | |
|---|---|
| Titanium dioxide A2 (component A) | 12 |
| (dimethicone-coated, D50 = 112 nm) | |
| Quaternium-90 bentonite (component B) | 0.35 |
| Dimethicone-coated yellow iron oxide (component C) | 1.9 |
| Dimethicone-coated red iron oxide (component C) | 0.6 |
| Dimethicone-coated black iron oxide (component C) | 0.3 |
| Dimethicone-coated alumina | 3 |
| Dimethicone-coated synthetic fluorphlogopite | 5 |
| Dimethicone/aluminum hydroxide-coated mica | 2 |
| White pigment titanium dioxide (component E) (aluminum hydroxide-coated, D50 = 250 nm, D90 = 390 nm, D10 =160 nm) | 5 |
| Amorphous silica (oil absorption: 60 ml/100 g) | 10 |
| Dimethicone (nonvolatile) | 13.5 |
| Phenyl trimethicone | 11 |
| Squalane | 1 |
| Tocopherol | 0.1 |
| Olive fruit oil | 1 |
| Jojoba seed oil | 1 |
| Isotridecyl isononanoate | 15 |
| Dicaprylyl carbonate | 5 |
| Triethylhexanoin | 3.3 |
| Sorbitan sesquiisostearate | 1.5 |
| Sorbeth-30 tetraoleate | 0.2 |
| Carnauba wax | 0.75 |
| Polyethylene wax | 6.5 |

### Formulation Example 9: Oily solid concealer

| | |
|---|---|
| Titanium dioxide A2 (component A) | 24 |
| (triethoxycaprylylsilane-coated, D50 = 112 nm) | |
| Quaternium-18 bentonite (component B) | 0.7 |
| White pigment titanium dioxide (component E) (triethoxycaprylylsilane-coated, D50 = 250 nm, D90 = 390 nm, D10 =160 nm) | 1.1 |
| Triethoxycaprylylsilane-coated yellow iron oxide (component C) | 4.8 |
| Triethoxycaprylylsilane-coated red iron oxide (component C) | 1 |
| Triethoxycaprylylsilane-coated black iron oxide (component C) | 0.2 |
| Dimethicone-coated synthetic fluorphlogopite | 9 |
| Dimethicone-coated amorphous silica (oil absorption: 60 ml/100 g) | 5 |
| Dimethicone-coated mica | 2 |
| Hydrogenated farnesene (volatile) (component D) | 4 |
| Hydrogenated polydecene | 12 |
| Diphenylsiloxy phenyl trimethicone | 10 |
| Triethylhexanoin | 12 |
| Diisostearyl malate | 2.5 |
| Sorbitan sesquiisostearate | 2.5 |
| Phytosteryl/octyldodecyl lauroyl glutamate | 1 |
| Sodium acetylated hyaluronate | 0.1 |
| Tocopherol | 0.1 |
| Polyethylene wax | 6 |
| Carnauba wax | 0.5 |
| Microcrystalline wax | 0.5 |

### Formulation Example 10: Oily cream foundation

| | |
|---|---|
| Titanium dioxide A1 (component A) | 8 |
| (dimethicone-coated, D50 = 109 nm) | |
| Quaternium-18 bentonite (component B) | 0.6 |
| White pigment titanium dioxide (component E) (dimethicone/alumina-coated, D50 = 250 nm, D90 = 390 nm, D10 =160 nm) | 5 |
| Dimethicone-coated yellow iron oxide (component C) | 2.5 |
| Dimethicone-coated red iron oxide (component C) | 0.45 |
| Dimethicone-coated black iron oxide (component C) | 0.25 |
| Dimethicone-coated mica | 8 |
| Hydrogen dimethicone-coated crystalline cellulose | 5 |
| Dimethicone (nonvolatile) | 10 |
| Isotridecyl isononanoate | 18.3 |
| Diphenylsiloxy phenyl trimethicone | 16 |
| (Dimethicone/vinyl dimethicone) crosspolymer | 4 |
| Dextrin palmitate | 9.5 |
| Polysilicone-15 | 9 |
| Phytosteryl/octyldodecyl lauroyl glutamate | 1.5 |
| Tocopherol | 0.1 |
| Sorbitan sesquiisostearate | 1.6 |
| Salicylic acid | 0.2 |

### Formulation Example 11: Oily cream eye shadow

| | |
|---|---|
| Titanium dioxide A1 (component A) (aluminum hydroxide-coated, D50 = 109 nm) | 4 |
| (Al/Mg) silicate (component B) | 0.3 |
| Dimethicone (nonvolatile) | 11 |
| Yellow iron oxide (component C) | 0.7 |
| Red iron oxide (component C) | 0.5 |
| Black iron oxide (component C) | 0.4 |
| White pigment titanium dioxide (component E) (aluminum hydroxide-coated, D50 = 250 nm, D90 = 390 nm, D10 = 160 nm) | 0.5 |
| Titanium dioxide-coated synthetic fluorphlogopite | 12 |
| Iron oxide-coated titanium mica | 1 |
| Synthetic fluorphlogopite | 24 |
| Mica | 3 |
| Diphenylsiloxy phenyl trimethicone | 9 |
| Diphenyl dimethicone | 4.1 |
| Polyglyceryl-2 triisostearate | 9 |
| Triethylhexanoin | 12 |
| Meadowfoam seed oil | 0.1 |
| Corn oil | 0.1 |
| Squalane | 0.5 |
| Sorbitan sesquiisostearate | 2.5 |
| Tocopherol | 0.1 |
| Ceresin | 3 |
| Sunflower seed wax | 3 |

### Formulation Example 12: Lip cream

| | |
|---|---|
| Titanium dioxide A1 (component A) (stearic acid/aluminum hydroxide-coated, D50 = 109 nm) | 7 |
| Disteardimonium hectorite (component B) | 0.35 |
| White pigment titanium dioxide (component E) | 0.5 |
| (aluminum hydroxide-coated, D50 = 250 nm, D90 = 390 nm, D10 = 160 nm) | |
| Barium Sulfate | 0.2 |
| Silica dimethyl silylate (16 nm) | 4 |
| Hydrogenated farnesene (volatile) (component D) | 17 |
| Hydrogenated polyisobutene | 28.6 |
| Diisostearyl malate | 18 |
| Polysilicone-15 | 9 |
| Bis-ethylhexyloxyphenol methoxyphenyl triazine | 1 |
| Polyglyceryl-2 triisostearate | 9 |
| Phytosteryl/octyldodecyl lauroyl glutamate | 4 |
| Yellow No. 4 | 0.7 |
| Red No. 201 | 0.2 |
| Red No. 202 | 0.1 |
| Dimethicone (nonvolatile) | 0.05 |
| Safflower oil | 0.1 |
| Jojoba seed oil | 0.1 |
| Tocopherol | 0.1 |

In Formulation Example 12, the total mass of particles having a minor axis length of 60 nm or less blended in the cosmetic was less than 5% by mass.

### Formulation Example 13: Oil-in-water type liquid base

| | |
|---|---|
| Zinc oxide A4 (component A) | 5.0 |
| (octyltriethoxysilane-coated, D50 = 110 nm) | |
| (Al/Mg) silicate (component B) | 0.6 |
| Octyltriethoxysilane-coated yellow iron oxide (component C) | 0.15 |
| Octyltriethoxysilane-coated red iron oxide (component C) | 0.03 |
| Octyltriethoxysilane-coated black iron oxide (component C) | 0.01 |
| Dipropylene glycol | 8 |
| 1,3-Butylene glycol | 2 |
| Glycerin | 1 |
| PEG-60 Hydrogenated castor oil | 1 |
| PEG-40 Hydrogenated castor oil | 2 |
| Polysilicone-15 | 9 |
| Alkyl benzoate (C12-15) | 7 |
| Ethanol | 6 |
| Ethylhexyl salicylate | 4.5 |
| Diethylamino hydroxybenzoyl hexyl benzoate | 4 |
| Amorphous silica (oil absorption: 60 ml/100 g) | 4 |
| Methylene bis-benzotriazolyl tetramethylbutylphenol | 3 |
| Bis-ethylhexyloxyphenol methoxyphenyl triazine | 3 |
| Ethylhexyl triazone | 2.5 |
| (C13-15) Alkane (volatile) (component D) | 8 |
| Dimethicone (nonvolatile) | 5 |
| Dicaprylyl carbonate | 1 |
| Trimethylsiloxysilicate | 1 |
| Decyl glucoside | 0.45 |
| Pentylene glycol | 2 |
| Phenoxyethanol | 0.3 |
| Isostearic acid | 0.2 |
| Lauroyl lysine-coated saccharomyces culture | 0.5 |
| (Acrylates/(C10-30) alkyl acrylate) crosspolymer | 0.08 |
| Carbomer | 0.10 |
| Xanthan gum | 0.06 |
| Sodium hydroxide | 0.05 |
| Sodium hyaluronate | 0.001 |
| Water | balance |

The present invention is of course not limited by these Formulation Examples, but has the scope as claimed in the appended claims. In addition, all blending amounts are expressed in mass percentage relative to the total mass of the cosmetic.

## Claims

1. An oily or emulsified makeup cosmetic comprising:
(A) metal oxide particles having a minor axis D50 of 90 to 140 nm, and a minor axis D90/D10 of 2.1 or less; and
(B) a clay mineral,
the makeup cosmetic being free of microplastic beads and volatile cyclic silicones.

2. The makeup cosmetic according to claim 1, wherein the metal oxide particles are contained in an amount of 2 to 30% by mass relative to 100% of a total mass of the makeup cosmetic.

3. The makeup cosmetic according to claim 1, wherein the clay mineral is contained in an amount of 0.25 to 5% by mass relative to 100% of a total mass of the makeup cosmetic.

4. The makeup cosmetic according to claim 1, further comprising (C) an iron oxide selected from the group consisting of yellow iron oxide, red iron oxide, and black iron oxide.

5. The makeup cosmetic according to claim 1, being of oily or water-in-oil type.

6. The makeup cosmetic according to claim 1, further comprising a white pigment titanium dioxide, wherein the white pigment titanium dioxide is contained in an amount of 5% by mass or less relative to 100% of a total mass of the makeup cosmetic.
